Europäisches Patentamt

**European Patent Office**

**Office européen des brevets**

(19)

(11) Publication number: **0 415 740 A1**

## EUROPEAN PATENT APPLICATION

(12)

(21) Application number: 90309450.6

(22) Date of filing: 29.08.90

(51) Int. Cl.⁵: **A61C 15/00**, D04H 1/44, D04H 1/54, A61K 7/16

(30) Priority: 30.08.89 GB 8919568

(43) Date of publication of application:
06.03.91 Bulletin 91/10

(84) Designated Contracting States:
AT BE CH DE DK ES FR GR IT LI LU NL SE

(71) Applicant: **DELRIMS LIMITED**
**7 Dalby Court, Gadbrook Business Centre**
**Northwich, Cheshire CW9 7TN(GB)**

(72) Inventor: **Stackhouse,Alan Linford**
**20 Bebington Road Higher Tranmere**
**Birkenhead Merseyside L42 6PU(GB)**

(74) Representative: **Lyons, Andrew John et al**
**ROYSTONS 531 Tower Building Water Street**
**Liverpool L3 1BA(GB)**

(54) **Materials for cleaning teeth.**

(57) A tooth cleaning aid is formed from a strip (10A, 10B) of material with a lattice pattern (12) which leaves pads (14) in relief.

The strip (10A, 10B) is treated with, for example, fluorides, bactericides, flavourings which are held in micro capsules (20A, 20B) until released by rubbing. The pads (14) are more absorbent which favours take up of the active ingredients.

FIG 2

EP 0 415 740 A1

## MATERIALS FOR CLEANING TEETH

This invention relates to materials for cleaning teeth, particularly for cleaning between teeth.

Dental floss is well known for cleaning between teeth, particularly in relation to dental plaque and its removal or control of tendency to form. Dental floss is a filamentary material that not everyone finds easy to use.

It is an object of this invention to provide an alternative that could be more attractive to those who do not like dental floss.

According to this invention material for cleaning between teeth is provided in strip form, i.e. wider than it is thick so that its thickness will pass between teeth but its width will engage more tooth surface than filamentary dental floss, and preferably having a tooth engaging surface presenting relatively high and low points or regions to aid scouring of the tooth surface to be cleaned.

Preferably,the relatively high points or regions of the strip material have resilience and/or absorbence, the former the further to aid scouring action and adaption to irregularities of tooth surfaces and the latter to aid taking up and removal of even the most troublesome components of dental plaque.

It is preferred that said relative high and low points or regions are imposed upon the strip by deformation thereof, for example achieved by impression accompanied by any necessary conditions, such as heat, required to make same effectively permanent.

One suitable strip material comprises a non-woven fibre web, say of synthetic plastics fibres of suitable toughness, conveniently polyester, that is relatively compressed at the low points or regions, say by a substantially permanent compressed formation, as may be done at least for spun-laid randomised polyester fibre webs or mats by passage between heated patterned rollers to impose localised heat bonds of relatively reduced permeability compared with pads then relatively free and upstanding between those heat bonds. Heat bonds may conveniently be in a lattice-like pattern.

Another preferred feature hereof is application at least to tooth-engaging surfaces of said strip materials of other useful or desirable materials, for example calcification-enhancing fluorides and/or foreign organism inhibiting bactericides and/or deodourising and mouth-freshening flavouring substances, and/or products capable of leaving on teeth a film or coating that temporarily resists and retards attachment and/or development of bacteria, that will release as the strip materials are rubbed against tooth surfaces. At least where the strip material has absorbence such other useful or desirable materials may be in liquid, perhaps even vapour, form but micro-encapsulated as droplets (or vapour captures) within reasonably readily frangible capsules, say of gelatins and/or gums as used in coascervation to produce an emulsion, typically in de-ionised water capable of being sprayed or printed onto the strip materials, whether individually or in larger pieces prior to cutting or stamping out required or desired strips.

All three of fluoride, bactericide and flavouring micro-capsules may be present in the same emulsion and in suitable proportions to suit requirements or preferences. Most often, at least for conventional concentrations of fluorides and bactericides, the proportions of such capsules would likely favour bactericide relative to flavour and flavour relative to fluoride.

If a bacterial attachment/development inhibitor or retarder is used, perceived need for bactericide may be reduced or even eliminated, and suitable products can be micro-encapsulated in combination with flavouring.

For lattice-bonded polyester fibrous strip materials micro-capsule sizes of 2 or 3 microns nominal diameter and making up about 20% of an emulsion in de-ionised water, and pads of about 0.25 to 1.00 millimetre square, have been found to be suitable.

A convenient working width of strip material hereof for tooth cleaning purposes is about 5 or 6 millimetres, and said working width may extend between wider tabs that can serve either or both of the purposes of aiding gripping by the user and of being rubbed over major surfaces of the teeth.

A convenient thickness for strip material hereof has been found to be about 0.25 millimetre nominal between high points or region on opposite major sides of a strip.

All of the aforesaid proportional, quantity and size indications should be taken as exemplary, not limiting.

Practical implementation of one embodiment of this invention will now be specifically described, by way of example, with reference to the accompanying diagrammatic drawing, in which:

Figure 1 is a plan view of strip material suitable for tooth-cleaning use;

Figure 2 is a plan view of another strip material for the same purpose;

Figure 3 is an outline sectional view on the line A-A of Figure 1 and B-B of Figure 2;

Figure 4 is an idealised fragmentary indication of coated-on other materials; and

Figure 5 is an idealised fragmentary indication of impregnated-in other materials.

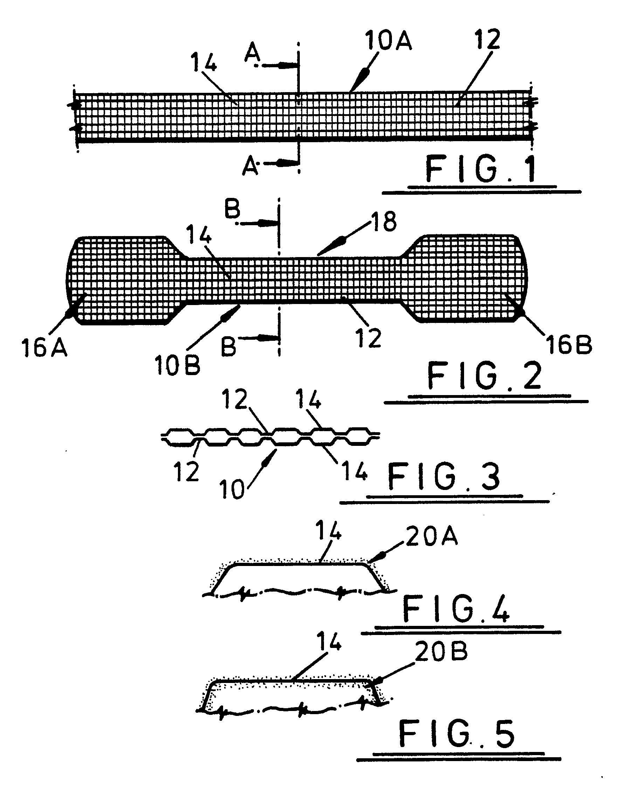

Referring first to Figures 1 and 3, a strip 10A of about 6 mm width is of spun-laid polyester fibre mat with a lattice 12 of heat bonding leaving relatively proud pads 14 of relatively free fibres exhibiting resilience and capable of a degree of interstitial absorbency or at least accommodation for micro-capsules, see dots at 20B in Figure 5. The strip 10A has a thickness of about 0.25 mm nominal between tops of pads 14 on its opposite major faces.

Figure 2 shows a variant tooth cleaning strip 10B with tabs 16A, 16B at each end of a working region 18 of the strip that is essentially as in Figure 1, hence repeating the same references 12 and 14 for the lattice bonding and the pads, respectively. Such a variant can be economically cut from larger sheet if the tabs are half the length of the working area and symmetrically wider, though other relative proportions can be used.

Tested polyester non-woven fibre mat or web material of nominal 0.25 mm thickness and lattice-bonded as above had pad areas of between 0.25 and 1.00 square millimetre and differential tensile strength about 2.5 times greater along the length of the strip 10 compared with transversely, actually in excess of 250 N/5 cm measured according to B.S. 2576, with tear strength at least 20N as measured according to B.S. 4303 and nearly twice as much transversely.

Separately micro-encapsulated fluoride, whether sodium fluoride or sodium monofluorophosphate at upto about two parts per million in suitable carrier fluid, for example isopropylinvictate, and bactericide, such as commercially available as Irgasan DP 300 at manufacturers' recommended concentration in similar carrier fluid, and flavouring such as peppermint or spearmint, conveniently all present together (in suitable proportions for the purposes hereof) in de-ionised water at about 20% thereof is readily applied to the strips 10. Such application can be by painting or printing processes, see Figure 4 for layer 20A, or pressure-jetting to assist penetration of the pads, see at 20B in Figure 5. In practice, most application procedures will result in micro-capsules of about 2-3 micron diameter achieving significant take-up within as well as upon the surfaces of the pads of the strips 10.

If a bacteria attachment/development retarder or inhibitor is used, it may be a suitable ester, for example IPM (isopropyl-myristate), and could replace bactericide, even be done in combination with flavouring in the same micro-capsules. Application would not be affected.

The actual pattern of bonding 12 of Figures 1 and 2 is not critical, for example non-parallel to the left-to-right lattice lines of those Figures, typically inclined by up to one pad width or more over the normal working area (see 18 of Figure 2), which may be achieved at cutting out or by canting the imposed bonding pattern itself relative to the high-tensile strength direction of the strip, or by a different lattice such as staggered or to give diamond shaped pads, or by a different pattern altogether, not necessarily isolating specifically identifiable pads but always providing some relief in the tooth-engaging surfaces thereof and preferably encouraging take up of micro-encapsulated useful other materials.

## Claims

1. A tooth cleaning aid comprising a strip wider than it is thick so that its thickness will pass between teeth but its width will engage more tooth surface than filamentary dental floss.

2. A tooth cleaning aid as claimed in claim 1 having a tooth engaging surface presenting relatively high and low points or regions to aid scouring of the tooth surface to be cleaned.

3. A tooth cleaning aid as claimed in claim 2, wherein the relatively high points or regions of the strip material have resilience.

4. A tooth cleaning aid as claimed in claim 2 or 3, wherein the relatively high points or regions have absorbence.

5. A tooth cleaning aid as claimed in claim 2, 3, or 4, wherein the relatively high and low points or regions are imposed upon the strip by deformation.

6. A tooth cleaning aid as claimed in claim 5 wherein deformation of the strip is achieved by impression.

7. A tooth cleaning aid as claimed in claim 6 wherein deformation of the strip is achieved by impression under heat.

8. A tooth cleaning aid as claimed in any one of the previous claims, wherein said strip comprises a non-woven fibre web.

9. A tooth cleaning aid as claimed in claim 8, wherein the strip is of spun-laid randomised plastics fibre.

10. A tooth cleaning aid as claimed in claim 9, produced by passing a strip of spun-laid randomised plastics fibre between heated patterned rollers, whereby localised heat bonds are imposed, these bonds of relatively reduced permeability compared with pads then relatively upstanding between said heat bonds.

11. A tooth cleaning aid as claimed in claim 10, wherein said heat bonds are in a lattice-like pattern.

12. A tooth cleaning aid as claimed in any one of the previous claims, wherein at least the relatively high points or regions have other useful or desirable materials applied to them.

13. A tooth cleaning aid as claimed in claim 12, wherein said useful or desirable materials are applied to the surface of at least the relatively high points.

14. A tooth cleaning aid as claimed in claim 12 or 13, wherein said useful or desirable materials are applied and taken up within as well as upon the surface of at least the relatively high points.

15. A tooth cleaning aid as claimed in claim 12, 13 or 14, wherein said useful or desirable materials are micro-encapsulated within frangible capsules to produce an emulsion.

FIG.1

FIG.2

FIG.3

FIG.4

FIG.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 450 849 (CERCEO) <br> * Column 1, line 48 - column 2, line 31; column 2, line 66 - column 3, line 4; column 3, lines 57-68; figures * <br> – – – | 1-3,5-7, 12 | A 61 C 15/00 <br> D 04 H 1/44 <br> D 04 H 1/54 <br> A 61 K 7/16 |
| Y | | 4,8,13,14 | |
| Y | GB-A-2 024 709 (KENDALL) <br> * Page 1, lines 60-62,77,78,97-125; figures * <br> – – – | 4,8 | |
| A | | 10,11 | |
| Y | DE-A-2 925 020 (WYBERT) <br> * Page 3, line 21 - page 4, line 11; figures * <br> – – – | 13,14 | |
| X | US-A-3 754 332 (WARREN) <br> * Column 2, lines 12-60; figures * <br> – – – | 1,2 | |
| A | | 3,4,12-14 | |
| X | US-A-3 491 776 (FLEMING) <br> * Column 1, line 63 - column 2, line 19; figures * <br> – – – | 1,2,12 | |
| A | | 13 | |
| X | US-A-4 270 556 (McALLISTER) <br> * Column 2, lines 14-55; figures * <br> – – – | 1,2,5 | TECHNICAL FIELDS SEARCHED (Int. Cl.5) |
| X | GB-A-6 916 92 (MARDER) <br> * Claim 1; figures * <br> – – – | 1 | A 61 C <br> D 04 H |
| A | GB-A-2 099 305 (WELSH) <br> * Page 1, lines 39-53; figure 4 * <br> – – – | 8,12-14 | |
| A | US-A-4 424 249 (RUPINSKAS) <br> * Column 1, line 58 - column 2, line 22; figure 1 * <br> – – – | 8-11 | |

–/–

The present search report has been drawn up for all claims

| Place of search | Date of completion of search | Examiner |
|---|---|---|
| The Hague | 22 October 90 | KLEIN C. |

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 142 538 (THORNTON)<br>* Column 2, lines 31-40; figures * | 10 | |
| A | US-A-4 034 771 (GUYTON)<br>* Abstract; figure * | 12,13 | |
| A | GB-A-2 059 266 (ILLINGWORTH) | | |
| A | DE-A-3 414 192 (BILCIURESCU) | | |
| A | EP-A-0 132 590 (LEX) | | |
| | | | **TECHNICAL FIELDS SEARCHED (Int. Cl.5)** |

The present search report has been drawn up for all claims

| Place of search | Date of completion of search | Examiner |
|---|---|---|
| The Hague | 22 October 90 | KLEIN C. |